# EUROPÄISCHE PATENTSCHRIFT

(11) **EP 0 966 433 B1**
(45) Veröffentlichungstag und Bekanntmachung des Hinweises auf die Patenterteilung: **22.05.2002**
(21) Anmeldenummer: 98912398.9
(22) Anmeldetag: 26.02.1998
(51) Int. Cl.: C07C 259/06

(54) **VERFAHREN ZUR HERSTELLUNG VON N-ARYL-N-HYDROXAMIDEN**
PREPARATION OF N-ARYL-N-HYDROXAMIDES
MODE DE PREPARATION DE N-ARYL-N-HYDROXAMIDES

(30) Priorität: 27.02.1997 DE 19707969
(43) Veröffentlichungstag der Anmeldung: 29.12.1999
(73) Patentinhaber: Consortium für elektrochemische Industrie GmbH, 81379 München (DE)
(72) Erfinder: STOHRER, Juergen, D-81479 München (DE)
(74) Vertreter: Fritz, Helmut, Dr.
(86) Internationale Anmeldenummer: EP9801084
(87) Internationale Veröffentlichungsnummer: WO9838159

(56) Entgegenhaltungen:
- I. C. CALDER AND P. J. WILLIAMS: "Preparation of N-Hydroxyphenacetin and formation of 1-acetyloxindoles from keten and N-phenylhydroxylamines" J. CHEM. SOC. CHEM. COMM., 1972, Seiten 891-892, XP002067094 in der Anmeldung erwähnt

## Beschreibung

Die Erfindung betrifft ein Verfahren zur Herstellung von N-Aryl-N-Hydroxamiden.

Verfahren zur Herstellung von N-Aryl-N-Hydroxamiden aus den entsprechenden N-Arylhydroxylaminen sind bekannt.
In der von Matlin, Stephen A.; Sammes, Peter G.; Upton, Roger M. in J. Chem. Soc., Perkin Trans. 1 (1979), (10), 2481-7 beschriebenen Darstellung werden N-Arylamide mit geeigneten Silylierungsmitteln wie z. B. Hexamethyldisilazan zu N-Trimethylsilyl-N-Arylamiden umgesetzt, die dann mit Peroxovanadat-Komplexen oxidiert werden. Aus den dabei entstehenden Vanadiumkomplexen der N-Aryl-N-Hydroxamide werden dann die gewünschten Produkte durch Auslaugung mit EDTA freigesetzt. Dieses Verfahren ist aufgrund der benötigten Schutzgruppen und der aufwendigen Aufarbeitung nicht kostengünstig.

In I. C. Calder, P. J. Williams, J. Chem. Soc. Chem. Comm. 1972, 891-892 ist die Umsetzung von Arylhydroxylaminen mit Keten beschrieben.
Dabei werden Arylhydroxylamine in Diethylether bzw. Chloroform mit Keten zur Reaktion gebracht. Das Produkt wird dabei jedoch nur in geringer Ausbeute (46%) und verunreinigt durch eine aufwendige basische Extraktion isoliert. Wird Keten im Überschuß verwendet, so entstehen zwei weitere Produkte (N,O-Diacetylarylhydroxylamin und 1-Acetyloxindol). Die Bildung des zweiten Nebenprodukts kann nur durch Durchführung der Reaktion bei -70°C unterdrückt werden.

In technischen Verfahren ist eine exakte stöchiometrische Dosierung von Keten schwierig durchzuführen, so daß nach dem bekannten Verfahren zur Herstellung von N-Aryl-N-Hydroxamiden zwangsläufig immer mindestens zwei Nebenprodukte vorliegen, die die Ausbeute vermindern und die Isolierung des Produkts erschweren.
Ferner können N-Aryl-Hydroxylamine, die ziehende Substituenten wie.z.B die Nitro-, Cyano- oder Halogengruppe besitzen, mit Ketenen auch bei stöchiometrischem Einsatz nicht selektiv zu N-Arylhydroxyamiden umgesetzt werden.

Die Zahl der Nebenprodukte kann in den beschriebenen Verfahren nur durch extrem tiefe und damit unwirtschaftliche Temperaturen verringert werden.

Ist ferner die Darstellung von N,O-Diacyl-Arylhydroxylaminen erwünscht, so fallen nach den bekannten Verfahren stets Nebenprodukte an, die nur durch Verfahren bei extrem tiefen und damit unwirtschaftlichen Temperaturen vermieden werden können.

Aufgabe der Erfindung war es daher, die Nachteile des Standes der Technik zu überwinden und ein kostengünstiges Verfahren zur Herstellung von N-Aryl-N-Hydroxamiden zu entwickeln, das sich durch hohen Umsatz, hohe Ausbeute und einfache Isolierung des Produktes auszeichnet und bei dem möglichst keine unerwünschten Nebenprodukte entstehen.

Ferner war es Aufgabe dieser Erfindung, ein kostengünstiges Verfahren zur Herstellung von N,O-Diacyl-Arylhydroxylaminen zu entwickeln, das sich durch hohe Ausbeute und einfache Isolierung des Produktes auszeichnet.

Diese Aufgabe wird durch das erfindungsgemäße Verfahren gelöst.

Gegenstand der Erfindung ist ein Verfahren zur Herstellung von N-Aryl-N-Hydroxamiden, bei dem Arylhydroxylamine (I)

Ar-NH-OH,

wobei Ar ein aromatischer Rest bedeutet,
mit Ketenen (II)

O=C=C(-R¹) (-R²),

wobei R¹ und R² gleich oder verschieden sind und Wasserstoff-, C₁ - C₁₂-Alkyl-Rest bedeuten,
zu N-Aryl-N-Hydroxamiden (III)

Ar-N(-OH)-C(=O)-CHR¹R²,

wobei Ar, R1 und R2 die oben angegebene Bedeutung haben,
umgesetzt werden, wobei keine Lösungsmittel verwendet werden, bei denen Acyl-Oxindole entstehen.

Arylhydroxylamine werden z.B. durch Reduktion von Nitrobenzol durch Zink, katalytisch durch Wasserstoff oder Hydrazin und durch elektrochemische Reduktion (siehe Houben-Weyl, Band 10/1, S. 1091ff. ) hergestellt.

Als Arylhydroxylamine werden vorzugsweise Arylhydroxylamine der Formel I verwendet 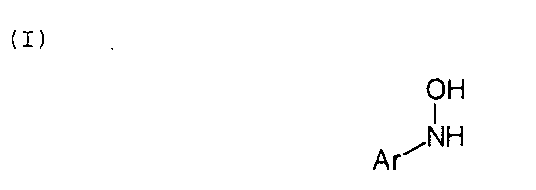 wobei Ar einen Arylrest ausgewählt aus der Gruppe Phenyl-, Biphenyl-, Naphthyl-, Pyridyl-, Pyrimidyl-, Pyridazinyl-, Triazinyl-, Furanyl-, Thiophenyl-, Pyrrolyl-, Thiazolinylrest bedeutet und
wobei dieser Arylrest durch eine oder mehrere, gleiche oder verschiedene Reste R³, ausgewählt aus der Gruppe Halogen-, Hydroxy-, Formyl-, Cyano-, Carbamoyl-, Carboxyrest, Ester oder Salz des Carboxyrests, Sulfonorest, Ester oder Salz des Sulfonorests, Sulfamoyl-, Nitro-, Nitroso-, Amino-, Phenyl-, Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-, Phospho-, Phosphono-, Phosphono-oxyrest, Ester oder Salz des Phosphonooxyrests substituiert sein können und
wobei Carbamoyl-, Sulfamoyl-, Amino- und Phenylreste unsubstituiert oder ein- oder mehrfach mit einem Rest R² substituiert sein können und die Aryl-C₁-C₅-alkyl-, C₁-C₁₂-Alkyl-, C₁-C₅-Alkoxy-, C₁-C₁₀-Carbonyl-, Carbonyl-C₁-C₆-alkyl-Reste gesättigt oder ungesättigt, verzweigt oder unverzweigt sein können und mit einem Rest R⁴ ein- oder mehrfach substituiert sein können, wobei
R⁴ gleich oder verschieden ist und Hydroxy-, Formyl-, Cyano-, Carboxyrest, Ester oder Salz des Carboxyrests, Carbamoyl-, Sulfono-, Sulfamoyl-, Nitro-, Nitroso-, Amino-, Phenyl-, C₁-C₅-Alkyl-, C₁ - C₅-Alkoxy, C₁ - C₅-Alkylcarbonylrest bedeutet
oder
je zwei Reste R³ oder R⁴ paarweise über eine Brücke [-CR⁵R⁶-]ₘ mit m gleich 0, 1, 2, 3 oder 4 verknüpft sein können und
R⁵ und R⁶ gleich oder verschieden sind und die für R³ genannten Bedeutungen haben und eine oder mehrere nicht benachbarte Gruppen [-CR²R³-] durch Sauerstoff, Schwefel oder einen gegebenenfalls einfach substituierten Iminorest und zwei benachbarte Gruppen [-CR⁵R⁶-] durch eine Gruppe [-CR⁵=CR⁶-] ersetzt sein können,
und
R¹ und R² gleich oder verschieden sind und Wasserstoff-, C₁ - C₁₂-Alkyl-Rest bedeuten.

Als Keten werden vorzugsweise Ketene der Formel (II) 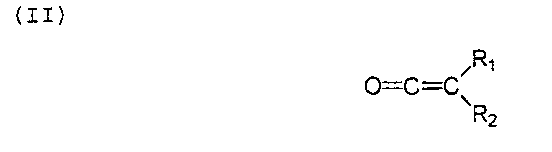 verwendet, wobei R¹ und R² die oben angegebene Bedeutung haben.

Die N-Aryl-N-Hydroxamide (III) haben die folgende Formel (III) 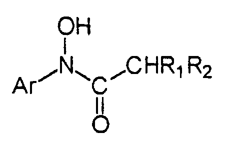 wobei Ar, R¹ und R² die oben angegebene Bedeutung haben.

Das erfindungsgemäße Verfahren erfolgt bevorzugt in Anwesenheit von Lösungsmitteln oder Lösungsmittelgemischen.

Das Wort Lösungsmittel bedeutet nicht, daß sich alle Reaktionskomponenten in diesem lösen müssen. Die Reaktion kann auch in einer Suspension oder Emulsion eines oder mehrerer Reaktionspartner durchgeführt werden.

Geeignete Lösungsmittel, in denen keine Acyl-Oxindole entstehen, sind z. B. Wasser, Alkohole wie Methanol, Ethanol, Isopropanol, Propanol, Butanol, Isobutanol, Tertiärbutanol, Hexanol, Cyclohexanol, Glycol, Methoxyethanol, Ester wie Methylacetat, Ethylacetat, Butylacetat, Cyclohexylacetat, Ethylformiat, Ethylbutyrat, Dimethylcarbonat, Säuren wie Essigsäure, Nitrile wie Acetonitril, Benzonitril, cyclische Ether wie Tetrahydrofuran, Dioxan, Amide wie Dimethylformamid, Dimethylacetamid, Sulfoxide wie Dimethylsulfoxid, aromatische Lösungsmittel wie Benzol, Toluol, Xylol, Chlorbenzol, Anisol, N,N-Dimethylanilin, Nitrobenzol, und heteroaromatische Verbindungen wie Pyridin, Pyrimidin sowie Gemische aus diesen Lösungsmitteln. Bevorzugte Lösungsmittel sind Wasser, Alkohole wie Methanol, Ethanol, Isopropanol, Propanol, Butanol, Isobutanol und Ester, wobei Methanol, Ethanol, Wasser und Ethylacetat besonders bevorzugt sind.

In Ethern wie Diethylether bzw. in halogenierten Kohlenwasserstoffen wie Chloroform entstehen Acyl-Oxindole, so daß diese beim erfindungsgemäßen Verfahren nicht verwendet werden.

Das erfindungsgemäße Verfahren erfolgt bevorzugt beim Druck der umgebenden Atmosphäre, also etwa 0.1 MPa (abs), es kann jedoch auch bei höheren oder niederen Drücken durchgeführt werden. Vorzugsweise wird das erfindungsgemäße Verfahren unter Ausschluß von Sauerstoff durchgeführt.

Vorzugsweise wird das erfindungsgemäße Verfahren bei Temperaturen von -30°C bis +100°C durchgeführt, besonders bevorzugt zwischen -10°C und +50°C.

Vorzugsweise werden zur Herstellung von N-Aryl-N-Hydroxamiden (III) 0.5 bis 2.5 mol Keten der Formel (II) pro mol Arylhydroxylamin der Formel (I) eingesetzt, besonders bevorzugt 1.0 bis 2.2.

Für die Herstellung von N,O-Diacylarylhydroxylaminen (IV) werden vorzugsweise 2.0 bis 3.0 mol Keten der Formel (II) pro mol Arylhydroxylamin der Formel (I) eingesetzt, wobei die N,O-Diacylarylhydroxylamine (IV) die Formel (IV) 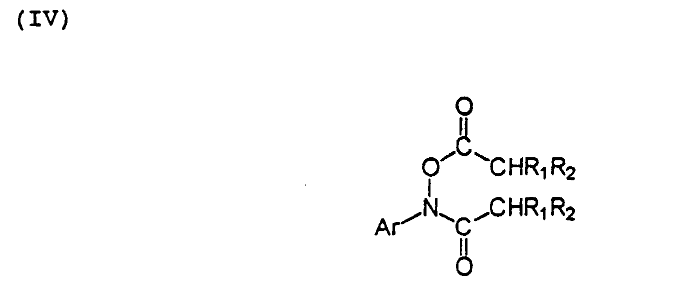 haben,wobei Ar, R¹ und R² die oben angegebene Bedeutung haben.

Beim erfindungsgemäßen Verfahren können, auch wenn nur N-Aryl-N-Hydroxamide (III) hergestellt werden sollen, als Nebenprodukt N,O-Diacylarylhydroxylamine (IV) anfallen, so daß für eine erwünschte hohe Ausbeute an N-Aryl-N-Hydroxamid N,O-Diacylarylhydroxylamin (IV) in das N-Aryl-N-Hydroxamid (III) umgewandelt werden muß.

Die Umsetzung von N,O-Diacylarylhydroxylamin (IV) in das N-Aryl-N-Hydroxamid (III) (im folgenden Rückspaltung genannt) erfolgt in der Gegenwart von basischen Verbindungen. Das N,O-Diacylarylhydroxylamin (IV) muß dazu nicht aus der Reaktionslösung isoliert werden.

Als basische Verbindungen, die die Rückspaltung bewirken, können in dem erfindungsgemäßen Verfahren verwendet werden:

stickstoffhaltige Basen ausgewählt aus der Gruppe Ammoniak, Amine wie Methylamin, Ethylamin, cyclohexylamin, Ethanolamin, Diethanolamin, Triethanolamin, Morpholin, Ethylendiamin, Trimethylamin, Triethylamin, Pyrrolidin, Piperidin, Anilin, Toluidin, Phenylendiamin, Hydrazin, Hydrazide wie Essigsäurehydrazid, Benzoesäurehydrazid, Hydroxylamin, Phenylhydroxylamin, Methylhydroxylamin, heterocyclische Stickstoffbasen wie Pyridin, Indol, Pyrrol, Imidazol, Pyrimidin,
oder
ionische Basen ausgewählt aus der Gruppe der
Alkali- und Erdalkalihydroxide, Alkali-, Ammonium- und Erdalkalicarbonate, der Alkalialkoholate, der Alkali- und Ammoniumsalze von Carbonsäuren, der Alkalisalze von Silanolen, der Alkali- und Ammoniumsalze von Thiolen, Thiophenolen und Xanthogensäuren, der Alkali- und Ammoniumsalze von Phenolen oder anderen aromatischen Hydroxyverbindungen
oder Mischungen aus diesen Basen.

Vorzugsweise werden bei dieser Umsetzung 0.001 bis 3 mol, besonders bevorzugt 0.05 bis 2 mol basische Verbindung pro mol Arylhydroxylamin der Formel (I) eingesetzt.

Die Umsetzung von N,O-Diacylarylhydroxylamin (IV) in das N-Aryl-N-Hydroxamid (III) erfolgt sinnvollerweise ohne Isolierung des Produktes in derselben Apparatur wie der erste Reaktionsschritt, sie kann jedoch auch zeitlich versetzt in anderen Apparaturen erfolgen.

Die Umsetzung erfolgt in Gegenwart der oben genannten Lösungsmittel und der oben genannten Temperaturen und Drücken.

Bei Anwendungen, in denen die Anwesenheit von N,O-Diacylarylhydroxylaminen (IV) nicht stört, kann auf die Rückspaltung verzichtet werden.

Die Produkte des erfindungsgemäßen Verfahren müssen nicht aus der Reaktionsmischung isoliert werden. Ist die Isolierung von N-Aryl-N-Hydroxamiden (III) gewünscht, so können sie durch Kristallisation oder Fällung aus den jeweiligen Reaktionslösungen erhalten werden oder durch Extraktion aus den Reaktionslösungen abgetrennt und nachfolgend kristallisiert werden.

Die erfindungsgemäßen Umsetzungen können auch mit Rohlösungen des Arylhydroxylamins durchgeführt werden, d. h. in Gegenwart von Nitro- und Nitrosoaromaten.

### Beispiele :

### 1.a) N-Acetyl-Phenylhydroxylamin

Eine Lösung von 10 g Phenylhydroxylamin in 100 ml THF wird bei 0°C unter intensiver Rührung über eine Fritte (A = 2cm², Porengröße 0) mit einem Gasstrom von Keten und Methan ( 100 mmol Keten/h; Ketenbestimmung durch Einleitung in NaOH-Lauge und Rücktitration) versetzt. Nach Einleiten von 1.0 Äquivalenten Keten wird durch DC auf Vollständigkeit der Umsetzung des Phenylhydroxylamins überprüft und gegebenenfalls weitere 0.05 Äquivalente Keten eingeleitet.
Nach HPLC liegen 95% als N-Hydroxyacetanilid vor und 5% als N,O-Bisacetylphenylhydroxylamin.
Nach vollständiger Umsetzung des Phenylhydroxylamins wird die Lösung unter Stickstoffatmosphäre mit 0.05 Äquivalenten an 25%igem wässrigem Ammoniak versetzt und 15 Minuten nachgerührt. Die Vollständigkeit der Rückspaltung wird durch DC überprüft und die Rückspaltung gegebenenfalls durch weiteren Zusatz an Ammoniak vervollständigt.
Nach DC liegt ausschließlich N-Hydroxyacetanilid vor.

### 1.b) N-Acetyl-Phenylhydroxylamin

Eine Lösung von 10g Phenylhydroxylamin in 100ml Essigsäureethylester wird bei 0°C unter intensiver Rührung über eine Fritte (A = 2cm², Porengröße 0) mit einem Gasstrom von Keten (200 mmol Keten/h; Ketenbestimmung durch Auswaage) versetzt. Nach Einleiten von 1.0 Äquivalenten Keten wird durch DC auf Vollständigkeit der Umsetzung des Phenylhydroxylamins überprüft und gegebenenfalls weitere 0.05 Äquivalente Keten eingeleitet.
Nach HPLC liegen 95% als N-Hydroxyacetanilid vor und 5% als N,O-Bisacetylphenylhydroxylamin.
Nach vollständiger Umsetzung des Phenylhydroxylamins wird die Lösung unter Stickstoffatmosphäre mit 0.05 Äquivalenten an 25%igem wässrigem Ammoniak versetzt und 15 Minuten nachgerührt. Die Vollständigkeit der Rückspaltung wird durch DC überprüft und die Rückspaltung gegebenenfalls durch weiteren Zusatz an Ammoniak vervollständigt.
Nach DC liegt ausschließlich N-Hydroxyacetanilid vor.

### 1.c) N-Acetyl-Phenylhydroxylamin

Eine Lösung von 10g Phenylhydroxylamin in 50ml 50%igem wässrigem Ethanol wird bei 10°C unter intensiver Rührung über eine Fritte (A = 2cm², Porengröße 0) mit einem Gasstrom von Keten und Methan (200 mmol Keten/h; Ketenbestimmung durch Einleitung in NaOH-Lauge und Rücktitration) versetzt. Nach Einleiten von 1.1 Äquivalenten Keten wird durch DC auf Vollständigkeit der Umsetzung des Phenylhydroxylamins überprüft und gegebenenfalls weitere 0.05 Äquivalente Keten eingeleitet.
Nach HPLC liegen 95% als N-Hydroxyacetanilid vor und 5% als N,O-Bisacetylphenylhydroxylamin.

Nach vollständiger Umsetzung des Phenylhydroxylamins wird die Lösung mit 0.05 Äquivalenten an 25%igem wässrigem Ammoniak versetzt und 15 Minuten nachgerührt. Die Vollständigkeit der Rückspaltung wird durch DC überprüft und die Rückspaltung gegebenenfalls durch weiteren Zusatz an Ammoniak vervollständigt.
Nach DC liegt ausschließlich N-Hydroxyacetanilid vor.

### 1.d) N-Acetyl-Phenylhydroxylamin

Durch katalytische Reduktion von Nitrobenzol wird eine Rohlösung von 87g Phenylhydroxylamin, 12g Anilin und 1g Azoxybenzol in 90%igem wässrigem Ethanol erhalten.
50ml dieser Lösung entsprechend 8.7g Phenylhydroxylamin werden bei 20°C unter intensiver Rührung über eine Fritte (A = 2cm², Porengröße 0) mit einem Gasstrom von Keten (100 mmol Keten/h; Ketenbestimmung durch Einleitung in NaOH-Lauge und Rücktitration) versetzt. Nach Einleiten von 1.1 Äquivalenten Keten wird durch DC auf Vollständigkeit der Umsetzung des Phenylhydroxylamins überprüft und gegebenenfalls weitere 0.05 Äquivalente Keten eingeleitet.
Nach vollständiger Umsetzung des Phenylhydroxylamins wird die Lösung unter Stickstoffatmosphäre mit 0.05 Äquivalenten an 25%igem wässrigem Ammoniak versetzt und 15 Minuten nachgerührt. Die Vollständigkeit der Rückspaltung wird durch DC überprüft und die Rückspaltung gegebenenfalls durch weiteren Zusatz an Ammoniak vervollständigt.

Nach HPLC liegen 86 % N-Hydroxyacetanilid, 12 % Acetanilid, und 0,7 % Azoxybenzol vor.

### 2.) N,O-Diacetyl-Phenylhydroxylamin

Eine Lösung von 10g Phenylhydroxylamin in 100ml THF wird bei 0°C unter intensiver Rührung über eine Fritte (A = 2cm², Porengröße 0) mit einem Gasstrom von Keten (100 mmol Keten/h; Ketenbestimmung durch Einleitung in NaOH-Lauge und Rücktitration) versetzt. Nach Einleiten von 2.0 Äquivalenten Keten wird durch DC auf Vollständigkeit der Umsetzung des Phenylhydroxylamins überprüft und gegebenenfalls weitere 0.05 Äquivalente Keten eingeleitet.
Nach HPLC liegt ausschließlich N,O-Diacetyl-Phenylhydroxylamin vor. Es wurde kein Acetyloxindol nachgewiesen.
Nach vollständiger Umsetzung des Phenylhydroxylamins wird die Lösung unter Stickstoffatmosphäre mit 1.05 Äquivalenten an 25%igem wässrigem Ammoniak versetzt und 30 Minuten nachgerührt. Die Vollständigkeit der Rückspaltung wird durch DC überprüft und die Rückspaltung gegebenenfalls durch weiteren Zusatz an Ammoniak vervollständigt.

Nach DC liegt ausschließlich N-Hydroxyacetanilid vor.

### 3.) N-Acetyl-4-cyanophenylhydroxylamin

Eine Lösung von 11.6g 4-Cyanophenylhydroxylamin in 100ml THF wird bei 0°C unter intensiver Rührung über eine Fritte (A=2cm², Porengröße 0) mit einem Gasstrom von Keten (100 mmol Keten/h; Ketenbestimmung durch Einleitung in NaOH-Lauge und Rücktitration) versetzt. Nach HPLC liegen nach Einleiten von 1.0 Äquivalenten Keten 45% als N,O-Diacetyl-4-cyanophenylhydroxylamin, 8% als N-Acetyl-4-cyanophenylhydroxylamin und 2% als O-Acetyl-4-cyanophenyl-hydroxylamin vor.
Nach Einleiten von insgesamt 1.8 Äquivalenten Keten wird durch DC auf Vollständigkeit der Umsetzung des 4-Cyanophenylhydroxylamins überprüft und gegebenenfalls weitere 0.05 Äquivalente Keten eingeleitet.
Nach vollständiger Umsetzung des 4-Cyanophenylhydroxylamins wird die Lösung mit 1.05 Äquivalenten an 25%igem wässerigem Ammoniak versetzt und 60 Minuten nachgerührt. Die Vollständigkeit der Rückspaltung wird durch DC überprüft und die Rückspaltung gegebenenfalls durch weiteren Zusatz an Ammoniak vervollständigt. Die Lösung wird von Lösungsmittel befreit, mit Methylenchlorid aufgenommen und mit 50ml 10% NaoH extrahiert. Die wässrige Phase wird abgetrennt und angesäuert. Der entstehende Niederschlag von N-Acetyl-4-cyanophenylhydroxylamin wird abfiltriert und getrocknet. (Ausbeute 13.8, 90%).

### 4.) N-Acetyl-2-methylphenylhydroxylamin

Eine Lösung von 10.9g 2-Methylphenylhydroxylamin in 100ml THF wird bei 0°C unter intensiver Rührung über eine Fritte (A=2cm², Porengröße 0) mit einem Gasstrom von Keten (100 mmol Keten/h; Ketenbestimmung durch Einleitung in NaOH-Lauge und Rücktitration) versetzt. Nach Einleiten von 1.0 Äquivalenten Keten wird durch DC auf Vollständigkeit der Umsetzung des 2-Methylphenylhydroxylamins überprüft und gegebenenfalls weitere 0.05 Äquivalente Keten eingeleitet.
Nach HPLC liegen 95% als N-Acetyl-2-methylphenylhydroxylamin vor und 5% als N,O-Bisacetyl-2-methylphenylhydroxylamin.
Nach vollständiger Umsetzung des 2-Methylphenylhydroxylamins wird die Lösung unter Stickstoffatmosphäre mit 0.05 Äquivalenten an 25%igem wässerigem Ammoniak versetzt und 15 Minuten nachgerührt. Die Vollständigkeit der Rückspaltung wird durch DC überprüft und die Rückspaltung gegebenenfalls durch weiteren Zusatz an Ammoniak vervollständigt.
Nach DC liegt ausschließlich
N-Acetyl-2-methylphenylhydroxylamin vor.

### 5.) N-Isobutyryl-Phenylhydroxylamin

Eine Lösung von 10g Phenylhydroxylamin in 100ml THF wird bei 0°C unter intensiver Rührung über eine Fritte (A=2cm², Porengröße 0) mit einem Gasstrom von Dimethylketen (100 mmol Dimethylketen/h; Bestimmung durch Einleitung in NaOH-Lauge und Rücktitration) versetzt. Nach Einleiten von 1.0 Äquivalenten Dimethylketen wird durch DC auf Vollständigkeit der Umsetzung des Phenylhydroxylamins überprüft und gegebenenfalls weitere 0.05 Äquivalente Dimethylketen eingeleitet.
Nach vollständiger Umsetzung des Phenylhydroxylamins wird die Lösung unter Stickstoffatmosphäre mit 0.05 Äquivalenten an 25%igem wässerigem Ammoniak versetzt und 60 Minuten nachgerührt. Die Vollständigkeit der Rückspaltung wird durch DC überprüft und die Rückspaltung gegebenenfalls durch weiteren Zusatz an Ammoniak vervollständigt.
Nach HPLC liegt ausschließlich N-Hydroxyisobutyrylanilid vor.

## Patentansprüche

1. Verfahren zur Herstellung von N-Aryl-N-Hydroxamiden, bei dem Arylhydroxylamine (I)
Ar-NH-OH,
wobei Ar ein aromatischer Rest bedeutet,
mit Ketenen (II)
O=C=C(-R¹) (-R²),
wobei R¹ und R² gleich oder verschieden sind und Wasserstoff-, C₁ - C₁₂-Alkyl-Rest bedeuten,
zu N-Aryl-N-Hydroxamiden (III)
Ar-N(-OH)-C(=O)-CHR¹R²,
wobei Ar, R1 und R2 die oben angegebene Bedeutung haben,
umgesetzt werden, **dadurch gekennzeichnet, daß** keine Lösungsmittel verwendet werden, bei denen Acyl-Oxindole entstehen.

2. Verfahren nach Anspruch 1, wobei N-Aryl-N-Hydroxamide (III) weiter mit Ketenen zu N,O-Diacylarylhydroxylaminen (IV) umgesetzt wird.

3. Verfahren nach Anspruch 1 oder2, **dadurch gekennzeichnet, daß** als Lösungsmittel kein Diethylether oder Chloroform verwendet werden.

4. Verfahren nach einem oder mehreren der Ansprüche 1 - 3, **dadurch gekennzeichnet, daß** N,O-Diacylarylhydroxylamine (IV) mit basischen Verbindungen zu N-Aryl-N-Hydroxamiden (III) selektiv rückgespalten werden.

## Claims

1. Process for the preparation of N-aryl-N-hydroxamides by reacting arylhydroxylamines (I)
Ar-NH-OH,
where Ar is an aromatic radical,
with ketenes (II)
O=C=C(-R¹) (-R²),
where R¹ and R² are identical or different and are hydrogen or a C₁-C₁₂-alkyl radical,
to give N-aryl-N-hydroxamides (III)
Ar-N(-OH)-C(=O)-CHR¹R²,
where Ar, R¹ and R² are as defined above,
**characterized in that** no solvents which form acyloxindoles are used.

2. Process according to Claim 1, wherein N-aryl-N-hydroxamides (III) are further reacted with ketenes to give N,O-diacylarylhydroxylamines (IV).

3. Process according to Claim 1 or 2, **characterized in that** the solvent used is not diethyl ether or chloroform.

4. Process according to one or more of Claims 1-3, **characterized in that** N,O-diacylarylhydroxylamines (IV) are selectively cleaved using basic compounds to give N-aryl-N-hydroxamides (III).

## Revendications

1. Procédé de préparation de N-aryl-N-hydroxamides, dans lequel des arylhydroxylamines (I)
Ar-NH-OH,
dans lesquelles Ar représente un radical aromatique, sont mises à réagir avec des cétènes (II)
O=C=C(-R¹) (-R²),
dans lesquels R¹ et R² sont identiques ou différents et représentent un hydrogène ou un radical alkyle en C₁-C₁₂,
pour donner lieu à des N-aryl-N-hydroxamides (III)
Ar-N-(-OH)-C(=O)-CHR¹R²,
dans lesquels Ar, R1 et R2 ont la signification indiquée ci-dessus,
**caractérisé en ce que** l'on n'utilise aucun solvant formant des acyloxindoles.

2. Procédé selon la revendication 1, dans lequel des N-aryl-N-hydroxamides (III) sont en outre mis à réagir avec des cétènes pour donner lieu à des N,O-diacylarylhydroxylamines (IV).

3. Procédé selon la revendication 1 ou 2, **caractérisé en ce que** l'on n'utilise pas l'éther diéthylique ou le chloroforme en tant que solvant.

4. Procédé selon l'une ou plusieurs des revendications 1 à 3, **caractérisé en ce que** des N,O-diacylarylhydroxylamines (IV) sont clivées sélectivement avec des composés basiques pour donner lieu à des N-aryl-N-hydroxamides (III).
